# EUROPEAN PATENT SPECIFICATION

(11) **EP 2 571 998 B1**
(45) Date of publication and mention of the grant of the patent: **07.06.2017**
(21) Application number: 10720259.0
(22) Date of filing: 19.05.2010
(51) Int. Cl.: C12Q 1/68, C12Q 1/70

(54) **METHOD AND KIT FOR IDENTIFYING COMPOUNDS CAPABLE OF INHIBITING HUMAN PAPILLOMA VIRUS REPLICATION**
VERFAHREN UND KIT ZUR IDENTIFIZIERUNG VON VERBINDUNGEN DIE DIE REPLIKATION VON HUMANEN PAPILLOMA VIREN VERHINDERN
PROCÉDÉ ET TROUSSE POUR L'IDENTIFICATION DE COMPOSÉS CAPABLES D'INHIBER LA RÉPLICATION DU PAPILLOMAVIRUS HUMAIN

(43) Date of publication of application: 27.03.2013
(73) Proprietor: Icosagen Cell Factory OÜ, 61713 Tartu maakond (EE)
(72) Inventor: USTAV, Mart, 50604 Tartu (EE); USTAV, Ene, 50604 Tartu (EE); GEIMANEN, Jelizaveta, 50703 Tartu (EE); PIPITS, Regina, 50708 Tartu (EE); ISOK-PAAS, Helen, 10112 Tallinn (EE); REINSON, Tormi, 50707 Tartu (EE); USTAV, Mart, Jr., 50604 Tartu (EE); LAOS, Triin, 80029 Pärnu (EE); ORAV, Marit, Saue 76506 Harjumaa (EE); SALK, Kristiina, 10138 Tallinn (EE); MÄNNIK, Andres, 50409 Tartu (EE); REMM, Anu, 50411 Tartu (EE)
(74) Representative: Kahu, Sirje
(86) International application number: PCT/EE2010/000010
(87) International publication number: WO 2011/144216

(56) References cited:
- WO-A1-01/96610
- WO-A2-01/77294
- CLARK P C ET AL: "A novel drug screening assay for papillomavirus specific antiviral activity", ANTIVIRAL RESEARCH, ELSEVIER BV, NL, vol. 37, no. 2, 1 February 1998 (1998-02-01), pages 97-106, XP002110672, ISSN: 0166-3542, DOI: DOI:10.1016/S0166-3542(97)00066-1
- BROWN CRAIG ET AL: "p53 represses human papillomavirus type 16 DNA replication via the viral E2 protein", VIROLOGY JOURNAL, BIOMED CENTRAL, LONDON, GB, vol. 5, no. 1, 11 January 2008 (2008-01-11), page 5, XP021038353, ISSN: 1743-422X
- KRISTIINA SALK: "Kõrge- ja madalariski inimese papilloomiviiruste replikatsioonimehhanismide uurimine erinevates rakuliinides", [Online] 1 January 2009 (2009-01-01), DISSERTATION,, PAGE(S) 1 - 55, XP009143286, Retrieved from the Internet: URL:http://www.ebc.ee/kaitsmised/2009/magi stritood_2009/Kristiina_Salk.pdf> page 49, paragraph 3.1.2
- KADAJA M ET AL: "Papillomavirus DNA replication - From initiation to genomic instability", VIROLOGY, ACADEMIC PRESS,ORLANDO, US, vol. 384, no. 2, 20 February 2009 (2009-02-20), pages 360-368, XP025914401, ISSN: 0042-6822, DOI: DOI:10.1016/J.VIROL.2008.11.032 [retrieved on 2009-01-13]

## Description

### TECHNICAL FIELD OF THE INVENTION

The present invention relates to the fields of virology, cell biology, cell culturing, and drug development. More particularly the invention provides a method for screening for anti-HPV substances and a kit for screening for anti-HPV substances.

### BACKGROUND OF THE INVENTION

The continuous interest to study the human papillomaviruses (HPV) has been generated from their association with specific human cancers. HPV infects basal proliferating cells of the epithelium and induces the formation of benign tumours. In some cases this infection may lead to progression and formation of malignant carcinomas. The complete papillomavirus virion constitutes a protein coat (capsid) surrounding a circular, doublestranded DNA organized into coding and non-coding regions. Eight early (E1-E8) open reading frames (ORFs) and two late (L1, L2) ORFs have been identified in the coding region of papillomaviruses. The early ORFs encode proteins involved in viral DNA replication during the establishment, in continuous maintenance state and late amplification (E1 and E2), in regulation of viral gene expression and chromosome tethering (E2), virus assembly (E4), immortalisation and transformation (E6 and E7/high-risk HPVs only). Late ORFs are activated only after cell differentiation and encode viral capsid proteins (L1 and L2). In the noncoding Upstream Regulatory Region (URR) the promoters, enhancer and other regulatory elements in addition to the replication origin are located.

The current view divides the papillomaviral life cycle into three stages. First, following initial entry into the cell nucleus in the basal layer of the epithelium, where the apparatus necessary for replication exists, the PV genome is amplified, viral DNA is synthesized faster than chromosomal DNA, the copy number raises (up to 50-300 copies per cell) (for review, see Kadaja M, Silla T, Ustav E, Ustav M. Papillomavirus DNA replication - from initiation to genomic instability. Virology. 2009 Feb 20;384(2):360-8.). The second stage represents stable replication of HPV DNA in S-phase synchronized with chromosomal replication and maintenance of viral DNA as extrachromosomal multicopy nuclear episomes as a result of segregation/partitioning of the viral genome into the daughter cells. At this stage only early genes are expressed and neither the synthesis of capsid proteins L1 and L2 nor virion assembly occurs. Early gene products provide transforming proteins that ensure clonal expansion of infected cells. If infected cells detach from the basal membrane and reach upper layers of the skin or mucosa, they stop dividing and start differentiation (keratinisation). It triggers onset of the third step, vegetative viral DNA replication during which a) viral DNA amplification is initiated again, and then b) late proteins are synthesized and viral particles assembled (for review, see Kadaja M, Silla T, Ustav E, Ustav M. Papillomavirus DNA replication - from initiation to genomic instability. Virology. 2009 Feb 20;384(2):360-8.).

Modelling of these replication stages in cells has been problematic in case of human papillomaviruses. Most of the tissue culture cells do not support any mode of HPV genomic replication. Attempts to get viral genomic DNA replication going from transfected plasmids of ß-papillomavirus types has completely failed in any keratinocyte cell lines or primary keratinocytes. Also, it has been difficult to generate reproducibly human cell lines that carry stable HPV replicating genomes, especially that of the "low risk"-HPV types. The stable replication of HPV episomes has been accomplished just by a handful of laboratories. The episomal state has been shown to be allowed only in the presence of feeders or in conditions of raft cultures. W12, a frequently used HPV-16 cell line, has originated from a patient sample, but while cultivating W12 cells in monolayer, integration events have been shown to take place instead of maintenance of the episomal state of the viral genome.

Nevertheless, the replication of HPV replication origin containing plasmids can be demonstrated in many different cell lines of different species in case the production of E1 and E2 proteins is provided from heterologous expression vectors. The main factor which restricts the replication to certain epithelial cells is therefore the availability of coordinated expression of cellular transcription factors for the transcription of the mRNAs for viral proteins.

The vaccines targeting HPV-16 and HPV-18 or HPV-6b, HPV-11, HPV-16 and HPV-18 have been developed and are becoming increasingly available in many countries. It should be considered as a great achievement in fighting against cervical cancers. However, it is not sufficient, because the vaccines target at best only for four subtypes of hundreds of papillomaviruses, including "high risk"-type of mucosal or cutaneous skin papillomaviruses. Additionally, it has been shown convincingly that HPV-16 and HPV-18 are prevalent viruses found in cervical carcinomas in developed countries. According to the molecular epidemiological analysis of the spread of the virus in developing countries, like in Sub-Saharan regions of Africa, other virus isolates like HPV-52 and HPV-35 are prevailing.

There is an urgent need for the small-molecule drugs, which can be used to block effectively the replication of the papillomavirus genome, therefore lowering the viral load per cell and avoiding the generation of viral particles and therefore the spread of the virus. However, this objective has been difficult to achieve due to the lack of an effective cellular system for screening for drug candidates. This cellular system should be compatible with the high-throughput and high-content format of the screening of the drug candidates and allow identifying the active substances in reproducible and cost-effective format. Animal xenograft models have been described previously by J. Duan, WO0040082 (A reproducible xenograft animal model for hosting and propagating human papillomavirus (HPV)), as well as primary keratinocytes are applied for hosting the viral genome by Kreider et al. 1993 and 1998, (US5541058, *In vitro* assay system for testing the effectiveness of anti-papilloma viral agents; US6200745, *Vitro* assay system using a human cell line for testing the effectiveness of anti-papilloma viral agents). However, these methods do not allow high-throughput screening for drug candidates, thus simpler and more convenient method would be required. Our group has previously discovered the ability of human osteosarcoma cell line U2OS to support the *in vitro* cultivation of HPV (K. Salk, 2009 Studies on the mechanisms of the DNA replication of high- and low-risk human papillomavirus in different cell lines. MSc thesis /in Estonian/; University of Tartu Press. Retrieved from the Internet: http://www.ebc.ee/kaitsmised/2009/magistritood_2009/Kristiina_Salk.pdf). However, maintenance of episomal HPV by itself is not sufficient for a high-throughput screening assay to identify possible HPV replication inhibitors.

The closest prior art, a method for identifying compounds capable of inhibiting HPV replication has been described by Clark P. R et al (Clark P. R., Roberts M.L., Cowsert L.M. "A novel drug screening assay for papillomavirus specific antiviral activity" Antiviral Research, Elsevier Science BV., vol. 37, no. 2, February 1998). SCC-4 cells supporting transient phase have been disclosed, and some cell lines have been reported to maintain 30-40 copies per cell and others 1-2 copies per cell. Clark P. R. et al does not disclose a cellular system that would be able to support all three replication phases of HPV-virus.

The components used in a method for identifying a compound capable of inhibiting HPV replication have been described by Brown C. et al (Brown C., Kowalczyk A.M., Taylor E.R., Morgan I.M., Gaston K. "p53 represses human papillomavirus type 16 DNA replication via the viral E2 protein" Virology Journal, Biomed Central, London, GB vol. 5 no 1, January 2008). The disclosed cell line supports only transient replication when co-transfected with a plasmid expressing E2m1, and does not support all replication phases of HPV.

### DISCLOSURE OF THE INVENTION

The present invention provides a method for identifying compounds capable of inhibiting Human Papillomavirus (HPV) DNA replication at initial amplificational replication, stable maintenance, or vegetative amplificational phase as well as a kit for identifying the compounds capable of inhibiting HPV DNA replication at initial amplificational replication, stable maintenance, or vegetative amplificational phase.

The present invention provides a method, wherein HPV genomic or subgenomic DNA is inserted into a cell line, wherein HPV DNA replication is supported, and further the influence of a compound on the HPV DNA replication is determined. The human osteosarcoma U2OS cell line was identified as a feasible host cell line to support HPV DNA replication. Now, according to the present invention, U2OS cells were identified as a suitable host for the propagation of genomes of mucosal and cutaneous tissue specific HPVs and for the HPV genome-related constructs. We have also demonstrated that amplificational replication of the HPV genome, resembling amplification in the vegetative phase of the viral life-cycle occurs, when HPV positive U2OScell clones are maintained in high density for extended periods with regular media (4-12≤ days).

Thus, a method is provided, wherein the quantitative detection of replicated HPV DNA or, more preferably, detection of a product of a reporter gene or a reaction product of a protein encoded by a reporter gene enables screening for factors inhibiting the HPV DNA replication at all different replication phases of HPV life cycle: a) the initial amplificational replication demonstrated by the transient replication assay; b) the stable HPV DNA replication, synchronous with cellular DNA replication, demonstrated by the analysis of low to high HPV-content subclones; and c) the amplificational replication resembling vegetative phase of the viral DNA replication. This kind of method can be widely used in pharmacological research and screening for new potential drug candidates for prevention or therapy of infections by various subtypes of HPVs.

The method for identifying compounds capable of inhibiting HPV DNA replication at initial amplificational replication, stable maintenance, or vegetative amplificational phase is provided comprising the following steps:
a. HPV DNA with complete or partial sequence enabling the transient, stable and vegetative replication steps of HPV DNA is introduced into a human osteosarcoma U2OS cell line enabling the transient, stable and vegetative replication of HPV DNA in these cells;
b. cell bank collections of stable subclones carrying extrachromosomal HPV DNA with different copy numbers per cell are generated;
c. chosen cell subclone (for HPV type or for copy number variations) is cultivated as a disperse monolayer culture of dividing cells and/or the chosen cell subclone is cultivated as a monolayer of dense culture;
d. the compound under investigation is applied to the cultivation vessel with the monolayer of the chosen subclone carrying the HPV DNA;
e. the presence or absence of the inhibitory effect of the compound on viral DNA maintenance and/or amplification in the cells is assessed;
f. if inhibitory effect on HPV DNA replication of a certain concentration of a compound is observed, the compound is identified as a candidate for HPV DNA replication inhibitors.

In preferred embodiment, the invention provides a method for identifying compounds capable of inhibiting HPV DNA latent replication, which comprises the following steps:
a. plasmid with complete or partial sequence of HPV DNA carrying all viral *cis-*sequences and *trans*-factors ensuring all steps of viral replication cycles, which may also encompass a sequence of a reporter gene, is introduced into human osteosarcoma cell line U2OS using methods like, but not limited to, electroporation or chemical transfection methods known in the art;
b. the clones of U2OS cell lines that carry extrachromosomally replicating HPV plasmids are isolated using selection markers providing resistance to the antibiotics like G418 or puromycin, or other selection markers known in the art;
c. the identified cell clones carrying different HPV copies per cell are grown up, the stability is determined and cell banks of these cell clones are generated;
d. the cells of the subclone selected for identification of HPV latent replication inhibitors are seeded at low density into 96 or 384 well plates, and cells are cultivated for a short period maintaining the HPV DNA replication in the latent phase;
e. subsequently, the compound under investigation is applied to the cell clone monolayer culture before confluency to identify inhibitors of latent replication;
f. the increase or lack of increase of the HPV copy number in the cells is determined by direct quantitative or semiquantitative measurement of the amount of viral DNA or by measurement of the amounts of the products of the reporter genes inserted into the HPV plasmid;
g. the compound is identified as a candidate for an inhibitor of HPV DNA latent replication, if inhibitory effect on HPV DNA stable replication of a certain concentration of the compound is observed.

In an another preferred embodiment, the invention provides a method for identifying compounds capable of inhibiting induced HPV DNA vegetative amplificational replication, which comprises the following steps:
a. plasmid with complete or partial sequence of HPV DNA carrying all viral *cis-*sequences and *trans*-factors ensuring all steps of viral DNA replication cycles, which may also encompass a sequence of a reporter gene, is introduced into human osteosarcoma cell line U2OS using methods like, but not limited to, electroporation or chemical transfection methods known in the art;
b. the clones of U2OS cell lines that carry extrachromosomally replicating HPV plasmids are isolated using selection markers providing resistance to the antibiotics like G418 or puromycin, or other selection markers known in the art;
c. the identified cell clones carrying different HPV copies per cell are characterized, their stability determined, amplification quantities measured and cell banks of these cell clones are generated;
d. the cells of the subclone selected for identification of vegetative amplificational replication are seeded into 96 or 384 well plates and let at confluency by additional feedings for at least 4 to 12≤ days for the launch of the exponential amplificational replication phase with increased copy number of the replicated episomal DNA per cell;
e. subsequently, the compound under investigation, the potential drug candidate, is added to the growth medium of the cultivation vessel of the U2OS cell clone monolayers at confluency to identify inhibitors of vegetative amplificational replication;
f. the increase or the lack of increase of the HPV copy number in the cells is determined by direct quantitative or semiquantitative measurement of the amount of viral DNA or by measurement of the amount of the products of the reporter genes inserted into the HPV plasmid;
g. the compound is identified as a candidate for an inhibitor of HPV DNA vegetative amplificational replication, if inhibitory effect on HPV DNA replication of a certain concentration of the compound is observed.

The inhibitory effect can be determined by any methods known in the art, which enables quantitative detection of the extrachromosomal (plasmid) DNA. However, most preferable methods comprise, but are not limited to, inserting nucleic acid sequences, which encode a reporter gene, to the episomally replicating construct. These reporter genes may encode any directly detectable and measurable proteins known in the art, or proteins catalyzing a reaction, product of which can be measured quantitatively or semiquantitatively, e.g by visual observation with a microscope. The measurable product may remain inside the cell or may be excreted into the media. Examples of such reporter genes comprise, but are not limited to, dGFP, luciferase, secreted alkaline phosphatase, Gaussia luciferase, dGFP-Luciferase fusion gene. Preferably, the nucleic acid sequence of the reporter gene is inserted to the region of HPV genome, which encodes for the L genes. Most preferably the nucleic acid sequence of the reporter gene substitutes the L1 and L2 genes of the HPV genome.

The subclones provided for selection from the generated cell banks are chosen from the ones carrying the variety of copy numbers ranging from low to high copy numbers of HPV plasmid per cell.

The subtypes of HPV provided in the present invention comprise, but are not limited to, HPV-18, HPV-16, HPV-6b, HPV-11, HPV-5 and HPV-8. These subtypes belong to mucosal high-risk, low-risk and cutaneous type of HPV subgroups, thus providing previously undescribed means for detecting substances capable for inhibiting the DNA replication of low-risk and skin-type of HPVs. The latent phase of HPV DNA replication provided in the invention, models the viral DNA replication process occurring in the dividing cells at the basal and suprabasal layer of the skin, infected by HPV. The vegetative amplificational replication phase of HPV replication provided in the invention models the viral DNA replication process occurring in nature in nondividing cells in the upper layers of the skin.

Moreover, the present invention provides a kit for identifying compounds capable of inhibiting HPV DNA replication at initial replication, stable maintenance and vegetative amplificational phase. This kit comprises at least: human osteosarcoma cell line U2OS, enabling initial replication, stable maintenance and vegetative amplificational phases of HPV DNA replication cycle; an extrachromosomally maintainable construct with complete or partial sequences of HPV DNA with L1 and L2 genes substituted with the reporter genes for introduction into the U2OS cell line; a compound or a library of compounds to be screened for anti-HPV activity; and a means for quantitative assessment of replicational, transcriptional or translational activity of HPV DNA in the cells. The extrachromosomally maintainable construct is according to SEQ ID NO:3.

Hereby, experimental data are provided to illustrate the ability of U2OS cell line to support HPV DNA replication at establishment, at latent maintenance phase as well as the unexpected phenomena of the induction of exponential viral DNA amplification mimicking the vegetative phase of the infection.

### DESCRIPTION OF THE FIGURES

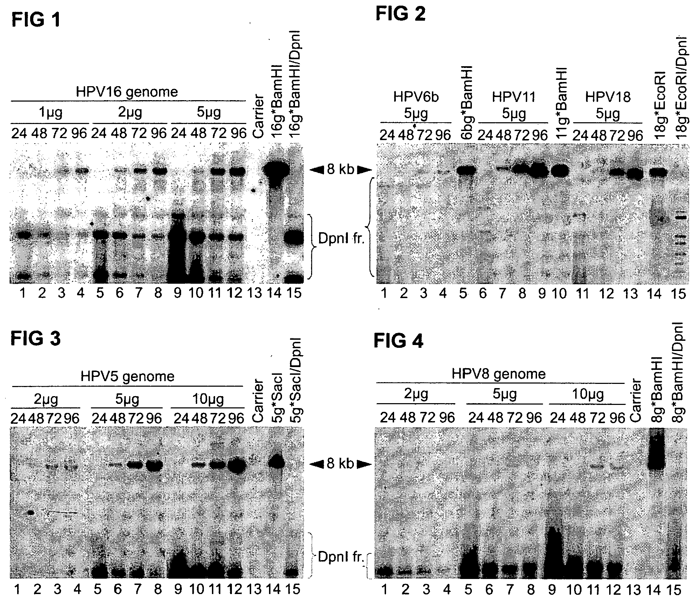

**Fig. 1** **-** **Fig. 4****. Transient DNA replication assays of mucosal high-risk, low-risk and cutaneous type of HPV subgroups.**
   U2OS cells were transfected with HPV-16 genome (Fig. 1), with HPV-6b, HPV-11, HPV-18 genomes (Fig. 2); with HPV-5 and HPV-8 genomes (Fig. 3 and Fig. 4) and short term replication assay was performed.
   Prior to transfection, the HPV DNAs were cleaved out from the vector backbone: HPV-18 genome from pBR322 vector with *Eco*RI; HPV-6b from pBR322 with *Bam*HI; HPV-16 and HPV-11 genomes from pUC19 with *Bam*HI; HPV-8 DNA from pUC9 vector with *Bam*HI; HPV-5 from pBR322 with *Sac*I. Linear HPV fragments (ca 8 kb) were religated at low DNA concentrations (5 µg/ml) for 16 hrs at 4°C.
   **Fig. 1****. Detection of dose response of the introduced mucosal type of HR-HPV16 reporter plasmid**: 1, 2, and 5 µg of religated circular plasmid DNA of the HPV-16 genome was introduced into U2OS cells. Low-molecular-weight DNA was extracted 24, 48, 72, and 96 hrs post transfection by Hirt lysis method and restriction analysis was performed using linearizing enzyme *Bam*HI and bacterial methylation sensitive *Dpn*I. For Southern blot hybridization the full length HPV-16 specific probe was used. The intensity of the linear 8 kb band increased in time (indicated by arrow), which is considered as the indication of replication of viral genome in these cells. Replication signals increased also concentration dependently.
   **Fig. 2****. Establishment of DNA replication from the LR-HPV-6b, LR-HPV-11 and HR-HPV-18.** Religated circular plasmid DNAs of HPV-6b, HPV-11 and HPV-18 genomes (5µg) were introduced into U2OS cells. The samples of Hirt lysis were digested with appropriate linearizing enzyme (look at markers) additionally to *Dpn*I, and the replicated HPV DNA signals were detected by Southern blotting with radiolabelled HPV genome-specific probes. The ca 8 kb linear *Dpn*I-resistant replication signals, which are increasing in time, are shown in case of all three investigated papillomavirus types.
   **Fig. 3****. Establishment of DNA replication from the cutaneous type of HPV-5 genome.** The religated circular plasmid DNA of HPV-5 genome was titrated (2, 5, 10 µg) into U2OS cells and Hirt lysis samples (episomal DNA, treated with *Sac*I/*Dpn*I) were loaded and viral DNA amplification was detected 24, 48, 72, 96 hrs post transfection by Southern blotting with full-length HPV-5 genomic probe (arrow).
   **Fig. 4****. Establishment of DNA replication from the cutaneous type of HPV-8 genome.** The religated circular plasmid DNA of HPV-8 genome was titrated (2, 5, 10 µg) into U2OS cells. The linear 8 kb bands of the replicated episomal DNA (*Bam*HI/*Dpn*I treated Hirt lysis samples) of HPV-8 genome, increasing in time and concentration dependently, are indicated by arrow.

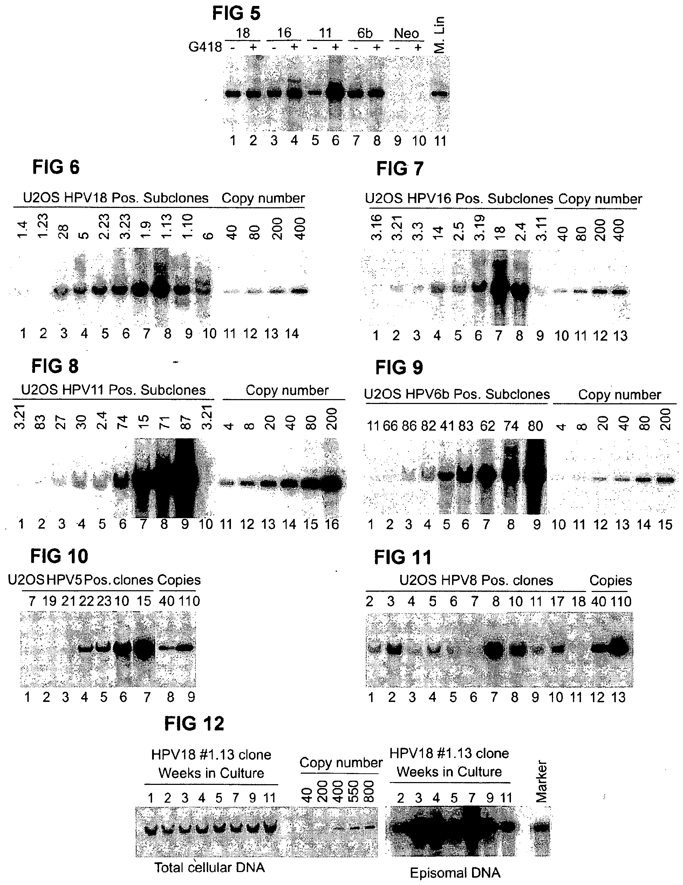

**Fig. 5** **-** **Fig. 12****. Stable maintenance of HPV genomes in U20S cells.**
   **Fig. 5****. Stable DNA replication of high- and low-risk HPV plasmids in U20S cells.** 5 µg of religated circular plasmid of HPV-6b, -11, -16, -18 together with 5 µg of AraD carrier DNA and with 2 µg of *Eco*0109I-linearized pNeo-EGFP plasmid were introduced into U2OS cells. The cells were put under G418 selection 48h after the transfection and were grown with selection about three weeks post-transfection. Low-molecular-weight extrachromosomal DNA samples from parental cell pools, extracted by Hirt method, were analysed. Samples were digested with linearizing enzyme and HPV signals were detected by Southern blotting with mixed radiolabelled HPV probes. DNA samples, which were cultivated 3 weeks without G418 selection post transfection, are also shown.
**Fig. 6** **-** **Fig. 12****. Southern blot analysis of single cell subclones of different HPV subtypes in U2OS cell line.** 5 µg of religated circular HPV plasmid together with 5 µg of carrier DNA (AraD) and 2 µg of linearized pNeo-EGFP or pBabeNeo plasmid was introduced into U2OS cells. Starting from 48 hrs after the transfection G418 selection was performed for about three weeks. Dilutions of 5000, 10 000 and 50 000 cells per 100 mm dish from the parental cell pools were transferred and single cell colonies were isolated, grown and analyzed. Total genomic DNA was isolated by standard method. 10 µg of linearized version of total cellular DNA was loaded on a gel and analyzed by Southern blotting with appropriate radiolabelled HPV genome-specific probe. Copy number was estimated by standard curves of marker lanes. Cell banks of these cell clones were generated.
   **Fig. 6****. Series of HR-HPV18 positive U2OS cell lines containing stable HPV-18 plasmids at different levels.** 10 µg of *Eco*RI-linearized total cellular DNA was analyzed by Southern blotting with radiolabelled full-length HPV-18 genome-specific probe. Clone numbers are indicated in the figure above the series and calculated copy numbers are shown by marker lanes. The identified cell clones carry different number of HPV-18 copies per cell.
   **Fig. 7****. Analysis of HR-HPV16 positive clonal cell populations.** 10 µg of *Bam*HI-linearized total cellular DNA was analyzed by Southern blotting with radiolabelled full-length HPV-16 genome-specific probe. Calculated copy numbers and clone numbers are indicated in the figure. The identified cell clones carry different number of HPV-16 copies per cell, varying from low to high copy number.
   **Fig. 8****. Series of LR-HPV11 positive U20S cell lines containing stable HPV-11 plasmids at different level of content.** 10 µg of *Bam*HI-linearized total cellular DNA was analyzed by Southern blotting with radiolabelled full-length HPV-11 genome-specific probe. Calculated copy numbers and clone numbers are indicated in the figure. The identified cell clones carry different number of HPV-11 copies per cell.
   **Fig. 9****. Series of LR-HPV6b positive U2OS cell lines containing stable HPV-6b plasmids at different levels.** 10 µg of *Bam*HI-linearized total cellular DNA was analyzed by Southern blotting with radiolabelled full-length HPV-6b genome-specific probe. Calculated copy numbers and clone numbers are indicated in the figure. The identified cell clones carry different number of HPV-6b copies per cell.
   **Fig. 10****. Human U2OS cell lines with low to high number of copies of stable HPV-5 plasmids**. 10 µg of *Sac*I-finearized total cellular DNA was analyzed by Southern blotting with radiolabelled full-length HPV-5 genome-specific probe. Calculated copy numbers and clone numbers are indicated in the figure. The identified cell clones carry different number of cutaneous type of HPV-5 copies per cell.
   **Fig. 11****. Human U2OS cell lines carrying low to high number of copies of stable HPV-8 plasmids per cell**. *Bam*HI-linearized total cellular DNA was analyzed by Southern blotting with radiolabelled full-length HPV-8 genome-specific probe. Calculated copy numbers and clone numbers are indicated in the figure. The identified cell clones carry different numbers of cutaneous type of HPV-8 copies per cell.
   **Fig. 12****. Maintenance of HPV-18 genome in U20S cell line**. HPV-18 #1.13 subclone was cultivated in regular monolayer cell culture conditions during next 11 weeks after the first detection of the positivity of HPV-18 signal. Stability of extrachromosomal HPV-18 DNA over the time course was determined by Southern blot analysis of linearized low-molecular weight DNA samples from Hirt lysates extracted from 100 mm culture dish. In parallel 2 µg of linearized total cellular DNA was loaded and HPV-18 maintenance signal compared during the same time course.

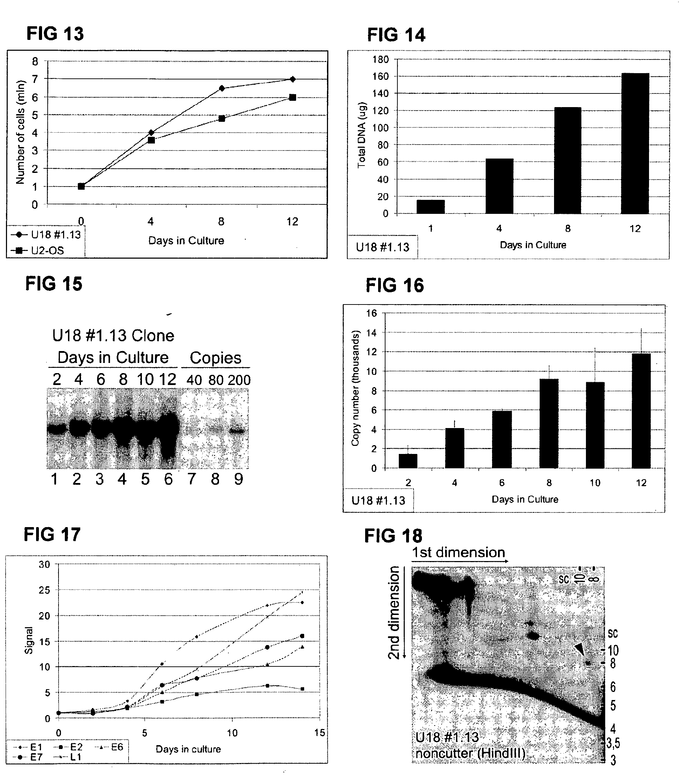

**Fig. 13** **-** **Fig. 18****. The induction of DNA amplification demonstrated by the HPV-18 positive cell line U18 #1.13.**
   A sample from U18 #1.13 cell line was taken from the cell bank, cells were grown as regular monolayers, and 10⁶ cells were seeded into each of the six 100 mm culture dishes for additional cultivation. 2 ml of fresh culture medium (IMDM) was added every two days, but no splitting of the cells was performed. Time points for analysis were taken the next day after adding the medium during 12 day growth period with 2-days interval. Time dependent growth series to obtain dense cell cultures are presented.
   **Fig. 13****. The growth curves of untransfected U2OS and HPV-18 positive cell line U18 #1.13.**
      Time dependent growth series to obtain dense cell cultures are presented. The cells were counted with Invitrogen Countess cell counter before analysis.
   **Fig. 14****. Amount of summarized total DNA in time series.**
      Total DNA was isolated by standard procedures, and DNA concentrations were measured by NanoDrop spectrophotometer ND-1000.
   **Fig. 15****. Southern blot analysis of the constant amount of total cellular DNA at different time points.** Equal amounts (shown 10 µg) of total cellular DNA were digested with linearizing enzyme *Eco*RI, and the amplification of HPV-18 genome was detected with radiolabelled HPV-18 genome-specific probe. The induction of DNA amplification is demonstrated.
   **Fig. 16****. Calculated HPV-18 copy numbers at different time points.**
      The replication signal intensities of U18 #1.13 cell line were measured using Phosphor-Imager and ImageQuant software. The HPV-18 genome copy number was estimated by standard curves of marker lanes. Three different series are summarized.
   **Fig. 17****. RT-PCR analysis of U18 #1.13 cell line mRNA levels at different time points.** mRNA levels of viral proteins were investigated at different time points during the induction of amplification. Total RNA was extracted with TRIzol reagent (Invitrogen) according to the manufacturer's protocol, and treated with DNase I (Fermentas) followed by heat inactivation of the enzyme. cDNA was synthesized with First Strand cDNA Synthesis kit (Fermentas) using 1 µg of total RNA as a template and oligo-dT primers in 20 µl reaction volume. cDNA was diluted into 160 µl and 2,5 µl of the dilution were used in a single PCR reaction along with 300 nM forward and reverse primers and 2 µl commercial master mix 5 x HOT FIREPol^{®} EvaGreen^{®} qPCR Mix (Solis Biodyne) in 10 µl of total reaction volume. Amplification was performed on 7900HT Real-Time PCR System (Applied Biosystems) and analyzed using comparative Ct (ΔCt) method, comparing HPV transcripts specific signals against reference gene β-actin signal. Signals were normalized to time point zero. RT-PCR analysis shows upregulation of the mRNA levels encoding viral proteins E1, E2, E6, E7, L1.
   **Fig. 18****. The neutral/neutral two-dimensional gel analysis (N/N 2D) for determining the structure of DNA replication intermediates (RIs).** The total DNA from U18 #1.13 cells grown as dense monolayer culture was analysed by digestion with *Hind*III enzyme as non-cutter for HPV-18 DNA, and separated on 2D gel. The sample of 10 µg of total DNA was loaded on a 0.4% agarose gel in 0.5x TBE buffer. The first dimension was electrophoresed at 10V for 48 hrs. The lane of interest was excised from the first dimension and rotated by 90°. 1% agarose gel in 0.5x TBE was run in the second dimension with EtBr (0.33 µg/ml) at 150V for 6 hrs. The DNA was transferred from the gel to a nylon filter, and probed with HPV-18 genome-specific probe. The size markers of supercoiled DNAs are shown in both directions. The presence of 8 kb circular plasmid is shown by arrow; the generation of high-molecular-weight plasmid multimers is also detected.

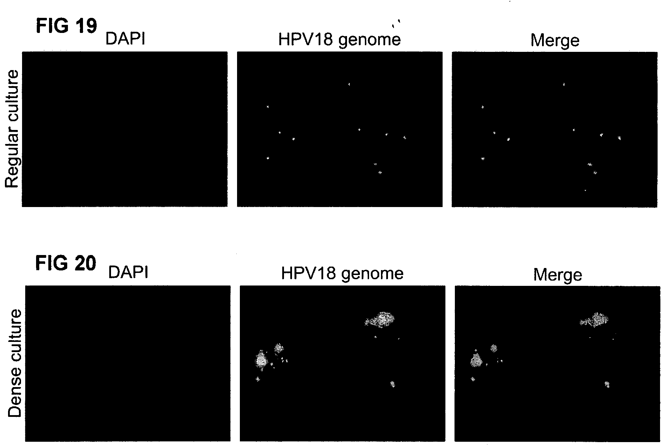

**Fig. 19** **-** **Fig. 20****. Increase in HPV-18 copy number in U2OS cells detected by fluorescence *in situ* hybridization.** 10⁶ cells of U18 #1.13 cell line were seeded into 100 mm culture dish, and grown for 2 weeks in cell culture, adding 2 ml of fresh culture medium in every two days, but no splitting of the cells was performed. Samples were collected on the first and on the 14th day after seeding, and analyzed by fluorescence *in situ* hybridization (FISH) (Invitrogen Corporation, TSA^{™} Kit #22). Hybridization probes were generated by nick translation, using HPV-18 genome as template and biotin-16-dUTP as label. Cell nuclei were counterstained with DAPI and mounted in PBS with 50% glycerol.
   **Fig. 19**. U18 #1.13 cells with HPV-18 signal on the first day after seeding.
   **Fig. 20**. U18 #1.13 cells with HPV-18 signal 2 weeks after seeding. The HPV-18 positive signal has increased in dense cell culture due to the amplification of viral genomes.
**Fig. 21****. The plasmid pUCHPV-18E, (SEQ ID NO 1.)**
   Most of the late region (L1 and L2 ORFs) of the HPV-18 genome was removed by cleavage with *Apa*I and *Bpi*I. The removed region was replaced with the fragment containing the sequences needed for propagation of the plasmid in *E. coli* cells (pMB 1 origin of replication and beta-lactamase resistance markergene (bla) amplified from pUC18 cloning vector). The inserted bacterial sequences can be removed by *Hind*III digestion.

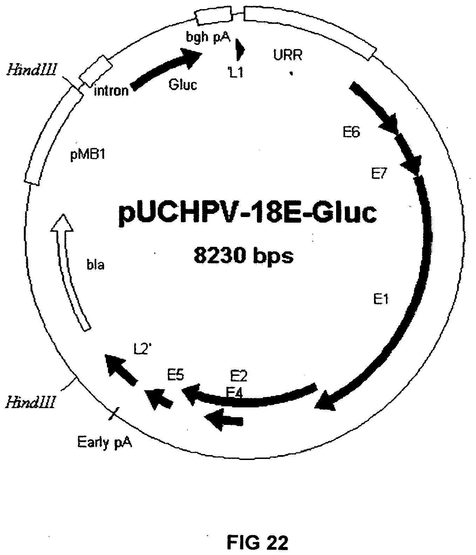

**Fig. 22****. The plasmid pUCHPV-18E-Gluc (SEQ ID NO 2).**
   Expression cassette that includes synthetic 5' intron element, codon optimised sequence encoding Gaussia luciferase marker gene, as well as bovine growth hormone polyadenylation signal, were inserted into the pUCHPV-18E so that the early region of the HPV-18 genome remained intact. The bacterial sequences can be removed by *Hind*III digestion.

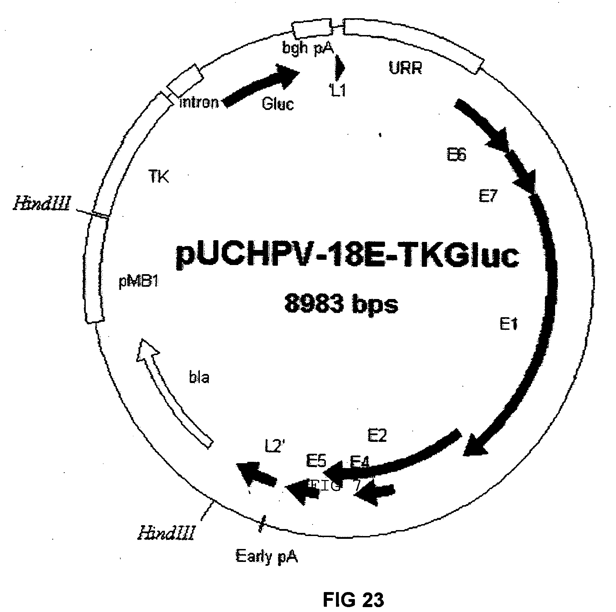

**Figure 23****. The plasmid pUCHPV-18E-TKGluc (SEQ ID NO 3).** The plasmid was made from the pUCHPV-18E-Gluc by insertion of the Herpes Simplex virus 1 (HSV 1) derived thymidine kinase (TK) promoter region in front of the Int-Gluc-bgh expression cassette. The bacterial sequences can be removed by *Hind*III digestion.

### DESCRIPTION OF THE EMBODIMENTS

### Example 1

### TRANSIENT HPV DNA REPLICATION IN U20S CELLS

Human papillomaviruses show strong tropism for epithelial cells. We have discovered that human osteosarcoma cell line U2OS, exhibiting epithelial adherent morphology, although derived from a moderately differentiated osteosarcoma, supported very effectively the HPV E1 and E2 protein dependent viral DNA replication, when the expression-vectors for viral replication proteins were used together with reporter plasmids containing viral origin. U2OS cells encode wild-type pRb and p53.

Hereafter we investigated, whether the viral *trans* factors (E1 and E2) could act in their native configurations supporting the replication of the viral genomes in U2OS monolayer cultures. A set of four different cutaneous type of papillomaviruses were included, two of them belonging to high-risk type (HR/HPV-18 and HR/HPV-16) and two to low-risk type (LR/HPV-11 and LR/HPV-6b) according to their prognosis for cancer development. Additionally, two subtypes, the HPV-5 and HPV-8 as skin infecting ß-papillomaviruses, were included. The U2OS cells were transfected with HPV-16 genome (Fig. 1), with HPV-6b, HPV-11, HPV-18 genomes (Fig. 2); with HPV-5 and HPV-8 genomes (Fig. 3 and Fig. 4, respectively) together with the carrier DNA (5µg of AraD plasmid) and short term replication assay was performed. Prior to transfection, the HPV DNAs were cleaved out from the vector backbone: HPV-18 genome from pBR322 vector with *Eco*RI; HPV-6b from pBR322 with *Bam*HI; HPV-16 and HPV-11 genomes from pUC19 with *Bam*HI; HPV-8 DNA from pUC9 vector with *Bam*HI; HPV-5 from pBR322 with *Sac*I. Linear HPV fragments (ca 8 kb) were gel-purified and religated at low DNA concentrations in the ligation mix (30µg/ml) for 16 hrs at 4°C.

As seen in Fig. 1, the introduction of increasing amounts (1, 2 and 5µg) of the HPV-16 plasmid into the U2OS cells raises the viral DNA replication signal up with increase in time (Fig. 1, lanes 1-4, 5-8, 9-12) and in concentration-dependent fashion (Fig.1, blocks of lanes 1-4; 5-8; 9-12). The same type short term transient replication pattern has been obtained in case of five other studied HPV types. As seen from the figures, the intensity of the linear 8 kb bands in the *Dpn*I-treated samples (indicated by arrows) increases in time, which is considered as the indication of replication of viral genome in these cells (Fig. 2, lanes 1-4 in case of 5 µg of inserted HPV-6b plasmid DNA, lanes 7-10 with HPV-11 and lanes 11-14 with HPV-18 DNA and Fig. 3 and ID for HPV-5 and HPV-8, respectively). All transfected HPV plasmids can initiate viral DNA replication in the U2OS cell line at quite comparable levels in short-term assays as has been observed in independent experiments.

The fact that the diverse groups of HPV circular genomes of HPV-6b, HPV-11, HPV-16, HPV-18, HPV-5 and HPV-8, respectively, are capable of establishing viral DNA replication in U2OS cells, suggests that the viral regulatory elements are adequately functional for supporting DNA replication of these virus types and that viral and cellular transcription and replication factors are adequately expressed. Thus, a compound capable for the inhibition of the first amplificational step of viral DNA replication in U2OS cell culture may be considered as a potential candidate for HPV treatment/prevention of HPV infection. The observation is valid at least for high-risk and low-risk mucosal HPVs as well as cutaneous HPVs.

### Example 2

### HPV STABLE REPLICATION IN U2OS MONOLAYER CULTURES

### Establishment of persistent HPV stable maintenance in U2OS cell line

Quite strong HPV genomic DNA replication signal in U2OS cells in transient assays suggested further evaluation of the capacity of HR- and LR-HPV plasmids for stable episomal replication. For this purpose we co-transfected into U2OS cells 5µg of HPV-6b, or HPV-11, HPV-16, HPV-18, HPV-5, HPV-8 circular plasmid together with 5µg AraD carrier DNA and with 2µg of *Eco*0109I -linearized of pNeo-EGFP or *Eco*RI-linearized pBabeNeo plasmid, encoding antibiotic resistance marker, which would allow the selection for the transfected cells. 48 hrs after the transfection G418 selection was performed. After two to three weeks of cultivation with G418 selection, the low-molecular weight (LMW) Hirt extracts from whole cell population ("*pool*" DNA) were analyzed by Southern blotting with radioactively labelled probes against the appropriate HPV types. The analysis shows that all tested samples contained HPV genomes at quite comparable levels, which indicates that the selected cells contained the HPV replicon (Fig. 5). The transfected HPV genomes were quite efficiently maintained even in series without selection (Fig. 1).

For the detection of cloned human cell lines that carry extrachromosomal replicating HPV episomes, dilutions of 5000, 10 000 and 50 000 cells per 100 mm dish were transferred from selected cell population and the single cell colonies were picked, expanded, and grown up under the G418 selection. Total genomic DNA was extracted from these clones and Southern blot analysis was performed with 10 µg of *Eco*RI-linearized (Fig. 6), *Bam*HI-linearized (Fig. 7, 8, 9, 11) or *Sac*I-linearized (Fig. 10) total cellular DNA using appropriate radiolabelled full-length HPV subtype-specific probes. Sets of single cell subclones for every different HPV type in U2OS cell line were detected (Fig. 6 - Fig. 11) and put into cell bank. In Fig. 6 and Fig. 7 positive examples of subclones of high-risk type of HPV-18 and HPV-16 are shown, carrying different copies of the HPV genomes per cell line. The U2OS cell clones carrying low-risk type of HPV-11 and HPV-6 were also isolated (shown in Fig. 8 and Fig. 9) as well as the subclones for ß-papillomavirus types HPV-5 and HPV-8 (shown in Fig. 10 and Fig. 11). The viral DNA copy number in different cell lines varied from very low to very high-copy per clone as indicated by Southern blotting. The copy number of the viral genomes was estimated using known quantities of the HPV plasmids on the same gel. Analysis of the episomal state of DNA plus FISH inspection was performed.

### Long term follow up of HPV-positive subclones by Southern blot analysis

For isolated HPV-positive subclones long term follow up was performed by Southern blot analysis to determine the stability of the episomal maintenance replication continuing into later passages. The majority of the tested cell lines were stable in monolayer cultures with regular cultivation conditions in monolayer cultures during at least two months of inspection (example with HPV-18 subclone #1.13 on Fig. 12). A certain loss of plasmids existed in low-risk type of HPV-11 and HPV-6b inspection, if continuous passage of the cell-lines took place.

HPV-18 #1.13 subclone was cultivated in regular monolayer cell culture conditions during 11 weeks starting from the detection of positive HPV-18 signal. The stability of extrachromosomal HPV-18 DNA over the time course was determined by Southern blot analysis of linearized (*Eco*RI) low-molecular weight DNA samples from Hirt lysates, extracted every time from one 100 mm culture dish. In parallel series equal amount (2 µg) of linearized cellular DNA (total DNA) was loaded and compared during the same time course. The HPV-18 full length genome specific probe was used.

The fact that the diverse group of HPV circular genomes of HPV-6b, HPV-11, HPV-16, HPV-18, HPV-5 and HPV-8, respectively, are capable of maintaining viral DNA replication in U2OS cells in monolayer cultures, further suggests that the viral regulatory elements are adequately functional for supporting at least stable or latent viral DNA replication step of these virus types and that viral and cellular transcription and replication factors are adequately expressed. Thus, a compound capable for the inhibition of the latent step of DNA replication in U2OS cell culture may be considered a potential candidate for HPV treatment/prevention in the latent phase of HPV infection. The observation is valid at least for high-risk and low-risk mucosal HPVs as well as cutaneous HPVs. The establishment of subclones with the HPV plasmid copy numbers varying from low to high confirms the usefulness of created tools, desired in the search for anti-HPV drugs.

### Example 3

### LATE AMPLIFICATION OF THE HPV GENOMES

### Genome amplification in a manner similar to differentiation-dependent viral amplification

In the productive stage of PV life cycle, amplification of the viral genome occurs in differentiated cells within the upper layer of epidermis. To study the productive stage of viral life cycle in tissue culture, the three-dimensional architecture of the epithelium has been usually tried to be reproduced with organotypic or raft cultures, suspension in methylcellulose, feeder cells, by using regulated culture and growth conditions.

We used an alternative method, only dense cell cultures to imitate differentiation-dependent viral amplification. For this purpose equal number of cells (for example 1 x 10⁶ cells per 10 cm culture dish) of appropriate HPV-positive cell clone were split on several dishes (for example 6) and maintained as regular confluent monolayers grown up to high densities. The total DNA or low molecular weight (Hirt) DNA samples were collected at day 2, 4, 6, 8, 10, 12, (...), isolated and analyzed.

Using the HPV-18 positive cell line H18 #1.13 as an example, the induction of HPV DNA amplification is shown in Fig. 13-18. The same type of amplification was tested for all HPV types under investigation. The examples of cell growth curves are given in Fig. 13 and the increasing amounts of total DNA extracted in series in Fig. 14. In Fig. 15, constant, equal amounts of total DNA from the series were loaded on the gel and analysed by Southern blot using *Eco*RI as a single cutter enzyme for HPV-18 and virus specific probe. The HPV DNA amplifies up at dense culture conditions (Fig. 15), shown in Fig. 16 with the quantitative data. Several repeated experiments were performed. RT-PCR analysis shows upregulation of synthesis of viral protein E1, E2, E6, E7, L1 RNA levels (Fig. 17). The neutral/neutral two-dimensional gel electrophoresis (2D) hybridization pattern indicates the presence of monomeric and multimeric forms of HPV-plasmids (Fig. 18). The differences in the shape of DNA replication intermediates in 2D restriction analysis at two stages (first and 12. day after seeding) would be the indication that the replication mode has been changed.

To characterize the appearance of intracellular HPV DNA episome formation supplementary to Southern blot analysis, the interphase and metaphase fluorescence *in situ* hybridization (FISH) was performed for studied subclones (Invitrogen Corporation, TSA^{™} Kit #22). Examples for interphase FISH for HPV-18 subclone #1.13 are shown in Fig. 19 - Fig. 20. The U18 #1.13 cells exhibit HPV-18 signal on the first day after seeding (Fig. 19). Two weeks after seeding the HPV-18 positive signal in U18 #1.13 cells has increased due to the amplification of viral genomes (Fig. 20).

As seen from these examples, HPV plasmid goes through an amplificational replication stage in confluent U2OS cells, bringing its copy number up to tens of thousands per cell, and therefore it is applicable for a person skilled in the art to use it in a high-throughput system for screening for agents exhibiting anti-HPV properties. Thus, a compound capable for the inhibition of amplificational DNA replication in U2OS cell culture may be considered a potential candidate for HPV treatment/prevention in the amplificational phase of HPV infection. The observation is valid at least for high-risk and low-risk mucosal HPVs as well as cutaneous HPVs.

### Example 4

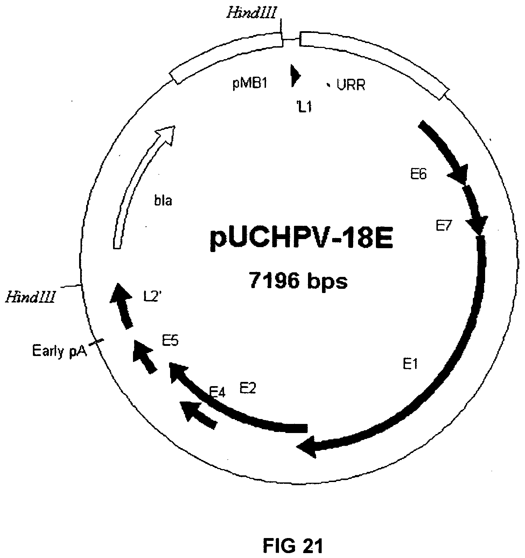

**The plasmid pUCHPV-18E, (SEQ ID NO 1.)** Most of the late region (L1 and L2 ORFs) of the HPV-18 genome was removed by cleavage with *Apa*I and *Bpi*I. The removed region was replaced with the fragment containing the sequences needed for the propagation of the plasmid in *E. coli* cells (pMB1 origin of replication and beta-lactamase resistance marker gene (bla) amplified from pUC18 cloning vector). The inserted bacterial sequences were removed by *Hind*III digestion. As a result, a plasmid construct with HPV-18 early region was obtained. The map of the plasmid is presented in Fig. 21.

### Example 5

**The plasmid pUCHPV-18E-Gluc (SEQ ID NO 2).** Expression cassette that includes synthetic 5' intron element, codon optimised sequence encoding Gaussia luciferase marker gene, as well as bovine growth hormone polyadenylation signal, were inserted into the pUCHPV-18E so that the early region of the HPV-18 genome remained intact. The bacterial sequences were removed by *Hind*III digestion. As a result, a plasmid with HPV-18 early region was constructed, which carries a reporter gene enabling quantitative or semi-quantitative detection of extrachromosomal high-risk mucosal HPV-18 DNA. The map of the plasmid is presented in Fig. 22.

### Example 6

**The plasmid pUCHPV-18E-TKGluc (SEQ ID NO 3).** The plasmid was made from the pUCHPV-18E-Gluc by insertion of the Herpes Simplex virus 1 (HSV 1) derived thymidine kinase (TK) promoter region in front of the Int-Gluc-bgh expression cassette. The bacterial sequences were removed by *Hind*III digestion. As a result, a plasmid with HPV-18 early region was constructed, which carries a TK promoter-regulated reporter gene enabling quantitative or semi-quantitative detection of extrachromosomal high-risk mucosal HPV-18 DNA. The map of the plasmid is presented in Fig. 23.

Examples 4-6 present a HPV-based construct, where L1 and L2 genes have been removed and replaced with a reporter gene. Accordingly, a useful instrument for quantitative or semi-quantitative assessment of the amount of replicated extrachromosomal DNA is provided.

### Example 7

A kit was completed by combining human osteosarcoma cell line U2OS, extrachromosomally maintainable HPV DNA plasmid pUCHPV-18E-TKGluc wherein the L1 and L2 genes are substituted with Gaussia luciferase marker gene. This construct was transfected into the U2OS cell line, the stable cell lines identified and cultivated to confluency. Any library of chemical compounds available or generated by a person skilled in the art can be applied to the preconfluent and/or confluent cell culture to screen the provided compounds from the library for their anti-HPV activity at stable maintenance and/or amplificational stage of viral DNA replication. The Gaussia luciferase reporter gene works as a means for quantitative or semi-quantitative assessment of replicated extrachromosomal DNA, as the amount of the fluorescent product of the inserted gene is readily detectable for a person skilled in the art either quantitatively by measuring the fluorescence or semi-quantitatively by visual observation with fluorescence microscope.

### Example 8

### A method for identifying compounds capable of inhibiting HPV DNA replication

Complete or partial sequence of HPV DNA carrying all necessary viral *cis*-sequences and *trans*-factors necessary for all steps of viral replication cycles was introduced into human osteosarcoma cell line U2OS using electroporation or chemical transfection methods know in the art. The clones of U2OS cell lines that carry extrachromosomally replicating HPV plasmids was isolated using selection marker providing resistance to G418. The identified cell clones carrying different HPV copies per cell were characterized, grown up and cell banks of these cell clones were generated. The cells of the subclone chosen for the identification of HPV latent replication inhibitors were seeded at low density into 96 well plates, drug candidates at different concentrations were added to the growth media, and cells were grown until confluent. Alternatively cells may be seeded into 384 well plates to increase the throughput. As another, preferred option, the cell culture was maintained for at least 5 to 7 days on the plates to become confluent, the potential drug candidates were added to the growth medium after cells had become confluent. The copy-number of HPV extrachromosomal copies was determined in the cells by direct differential measurement of the viral DNA in the cells or using reporters. Subsequently, the compound under investigation was applied to the cultivation vessel of the U2OS cell clone monolayers; the presence or absence of the inhibitory effect of the compound on viral DNA stable or amplificational replication in the cells was assessed by measuring the amount of the product of the reporter gene or the amount of extrachromosomal DNA; finally the compound was identified as a candidate for HPV DNA replication inhibitor, if inhibitory effect on HPV DNA replication of a certain concentration of the compound at certain copy number level at certain growth phase is observed at certain growth conditions.

## Claims

1. A method for identifying compounds capable of inhibiting Human Papilloma Virus (HPV) replication at initial amplificational replication, stable maintenance, or vegetative amplifictational phase, said method comprising following steps:
a. introducing HPV genomic or subgenomic DNA into a human osteosarcoma U2SO cell line enabling initial replicational, stable maintenance and late amplificational replication phases of HPV DNA replication cycle;
b. generating a collection of stable single cell subclones carrying extrachromosomal HPV DNA at different copy numbers per subclone;
c. cultivating cells of selected subclones as dispersed and/or dense monolayer cultures with regular media;
d. applying the compound under investigation to the cultivation vessel with the monolayer of the subclone of cell line carrying the HPV DNA;
e. assessing presence or absence of inhibitory effect of the compound on viral DNA maintenance and/or amplification in the cells;
f. if inhibitory effect on HPV DNA replication of a certain concentration of a compound is observed, the compound is identified as a candidate for HPV DNA replication inhibitor.

2. The method according to Claim 1, wherein the compound under investigation is applied to the cell subclone monolayer before obtaining confluency; and the HPV inhibitor is aimed to inhibit the latent phase of HPV DNA replication.

3. The method according to any of Claims 1 or 2, wherein the culture of the subclone is maintained by the consecutive passages at confluency for at least 4 to 12 days until the vegetative amplificational replication phase of the extrachromosomal HPV DNA launches and wherein the compound under investigation is applied to the medium of the cell clone monolayer at confluency, and wherein the HPV inhibitor is tested for inhibition of the vegetative amplificational phase of HPV DNA replication.

4. The method according to any one of Claims 1-3, wherein the presence or absence of the inhibitory effect is assessed by measuring quantitatively or semi quantitatively the amount of extrachromosomal viral DNA.

5. The method according to any of Claims 1-3, wherein a sequence of a reporter gene is inserted to the subgenomic fragment of the HPV DNA.

6. The method of Claim 5, wherein the sequence of the reporter gene substitutes the L1 and L2 genes of HPV.

7. The method of Claim 5, wherein the reporter gene is d1GFP, luciferase, secreted alkaline phosphatase (SEAP) or Gaussia luciferase.

8. The method of Claim 5, wherein the amount of the protein encoded by the reporter gene is measured.

9. The method of Claim 5, wherein the product of the reaction catalysed by the protein encoded by the reporter gene is measured.

10. The method according to any of Claim 1-3, wherein the HPV is high-risk mucosal HPV belonging to the subtype HPV-18 or HPV-16, or any other high-risk mucosal HPV.

11. The method of any of claims 1-3, wherein the HPV is low-risk mucosal HPV belonging to the subtype HPV-6b or HPV-11, or any other low-risk mucosal HPV.

12. The method of any of claims 1-3, wherein the HPV is cutaneous type of HPV belonging to the subtype HPV-5 or HPV-8, or any other cutaneous HPV.

13. A kit for identifying compounds capable of inhibiting HPV replication at initial replication, stable maintenance or vegetative amplificational phase, said kit comprising at least:
a) human osteosarcoma cell line U20S enabling initial replication, stable maintenance and vegetatitve amplificational phases of HPV DNA replication cycle;
b) an extrachromosomally maintainable construct with complete or partial sequence of HPV DNA with L1 and L2 genes substituted with reporter genes for introduction into the U20S cell line;
c) a compound or a library of compounds to be screened for anti-HPV activity;
d) a means for quantitative assessment of replicational, transcriptional or translational activity of HPV DNA in the cells.

14. The kit of claim 13, wherein the extrachromosomally maintainable construct is according to SEQ ID NO:3.

## Patentansprüche

1. Verfahren zum Identifizieren von Verbindungen, welche die Replikation der humanen Papillomiren (HPV) bei der ursprünglichen amplifizierenden Replikation, der Stabilhaltung oder in der vegetativen Amplifizierungsphase inhibieren können, wobei das Verfahren folgende Schritte umfasst:
a. Das Schleusen der HPV-genomischen oder -subgenomischen DNA in die menschliche Osteosarkom-Zelllinie U2SO, wodurch die ursprüngliche Replikation, die Stabilhaltung und die späteren amplifizierenden Replikationsphasen des DNA-Replikationszyklus von HPV ermöglicht werden;
b. Erzeugen einer Ansammlung von stabilen Einzelzell-Subklonen, welche an unterschiedlichen Kopien pro Subklon eine extrachromosomale DNA von HPV tragen;
c. Kultivierung von Zellen der ausgewählten Subklone als dispergierte und/oder dichte Einschichtkulturen in einem gewöhnlichen Nährmedium;
d. Das Geben der Verbindung der Untersuchung in den Ansatzbehälter mit der Einschichtkultur des Subklons der Zelllinie, welche die DNA von HPV trägt;
e. Nachweisen, ob die Hemmwirkung der Verbindung in Bezug auf das Erhalten und/oder Amplifikation der viralen DNA vorhanden ist oder fehlt;
f. Falls die Hemmwirkung einer bestimmten Konzentration der Verbindung in Bezug auf die DNA-Replikation von HPV festgestellt werden konnte, wird die Verbindung als ein möglicher DNA-Replikationshemmer von HPV identifiziert.

2. Verfahren nach Anspruch 1, **dadurch gekennzeichnet, dass** die Verbindung der Untersuchung in die Einschichtkultur des Zellsubklons gegeben wird bevor die Konfluenz erreicht ist; und der HPV-Hemmer ist dazu vorgesehen, die latente Phase der DNA-Replikation von HPV zu hemmen.

3. Verfahren nach einem der Ansprüche 1 oder 2, **dadurch gekennzeichnet, dass** die Kultur der Subklone durch nachfolgende Durchgänge bei Konfluenz innerhalb von wenigstens 4 bis 12 Tagen erhalten wird, bis die vegetative amplifizierende Replikationsphase der extrachromosomalen DNA von HPV anläuft, wobei die Verbindung der Untersuchung in das einschichtige Medium des Zellklons während der Konfluenz gegeben wird, und wobei der HPV-Hemmer in Bezug auf die Inhibition der vegetativen Amplifikationsphase der DNA-Replikation von HPV getestet wird.

4. Verfahren nach einem der Ansprüche 1-3, **dadurch gekennzeichnet, dass** die Bewertung des Vorhandenseins oder des Fehlens der Hemmwirkung durch die quantitative oder semiquantitative Messung der Anzahl der extrachromosomalen viralen DNA geschieht.

5. Verfahren nach einem der Ansprüche 1-3, **dadurch gekennzeichnet, dass** in das Subgenom-Fragment der DNA von HPV eine Sequenz des Reporter-Gens eingeführt wird.

6. Verfahren nach Anspruch 5, **dadurch gekennzeichnet, dass** die Sequenz des Reporter-Gens die L1- und L2-Gene von HPV substituiert.

7. Verfahren nach Anspruch 5, **dadurch gekennzeichnet, dass** das Reporter-Gen d1GFP, Luziferase, sekretorische alkalische Phosphatase (SEAP) oder Gaussia Luziferase ist.

8. Verfahren nach Anspruch 5, **dadurch gekennzeichnet, dass** die Anzahl des durch das Reporter-Gen kodierten Proteins gemessen wird.

9. Verfahren nach Anspruch 5, **dadurch gekennzeichnet, dass** das Reaktionsprodukt gemessen wird, das vom durch das Reporter-Gen kodierte Protein katalysiert wurde.

10. Verfahren nach einem der Ansprüche 1-3, **dadurch gekennzeichnet, dass** der HPV zum mukosalen HPV-Hochrisikotyp des Subtyps HPV-18 oder HPV-16 oder zu einem anderen mukosalen HPV-Hochrisikotyp gehört.

11. Verfahren nach einem der Ansprüche 1-3, **dadurch gekennzeichnet, dass** der HPV zum mukosalen HPV-Niedrigrisikotyp des Subtyps HPV-6b oder HPV-11 oder zu einem anderen mukosalen HPV-Niedrigrisikotyp gehört.

12. Verfahren nach einem der Ansprüche 1-3, **dadurch gekennzeichnet, dass** der HPV zum kutanen HPV-Typ des Subtyps HPV-5 oder HPV-8 oder zu einem anderen kutanen HPV gehört.

13. Kit zum Identifizieren von Verbindungen, welche die Replikation von HPV bei der ursprünglichen Replikation, der Stabilhaltung oder in der vegetativen Amplifizierungsphase inhibieren können, wobei das Kit mindestens Folgendes umfasst:
a) menschliche Osteosarkom-Zelllinie U20S, welche die Phasen der ursprünglichen Replikation, der Stabilhaltung und der vegetativen Amplifizierung des DNA-Replikationszyklus von HPV ermöglicht;
b) ein extrachromosomalerhaltbares Konstrukt mit vollständiger oder partieller Sequenz der DNA von HPV mit L1- und L2-Genen, welche mit Reporter-Genen subsituiert sind, zum Einführen in die U20S-Zelllinie;
c) eine Verbindung oder eine Bibliothek der Verbindungen, welche auf die Anti-HPV-Aktivität geprüft wird;
d) ein Mittel zur quantitativen Bewertung der Replikations-, der Transkriptions- oder der Translationsaktivität der DNA von HPV in den Zellen.

14. Kit nach Anspruck 13, **dadurch gekennzeichnet, dass** der extrachromosomalerhaltbares Konstrukt der SEQ ID NO:3 entspricht.

## Revendications

1. Le procédé pour identifier des composés capables d'inhiber la réplication du virus du papillome humain (VPH) lors d'une réplication amplificatrice initiale, d'une maintenance stable, ou d'une phase amplificatrice végétative, ledit procédé comprenant les étapes suivantes :
a. l'introduction de l'ADN génomique ou subgénomique du VPH dans une lignée cellulaire U2SO d'ostéosarcome humain permettant une réplication initiale, une maintenance stable, et des phases de réplication amplificatrices tardives du cycle de réplication de l'ADN du VPH ;
b. la génération d'une collection de sous-clones stables de cellules individuelles portant de l'ADN du VPH extrachromosomique à différents nombres de copies par sous-clone ;
c. la culture des cellules de sous-clones sélectionnés en tant que cultures monocouches dispersées et / ou denses avec des milieux réguliers ;
d. l'application du composé à l'étude dans le récipient de culture avec la monocouche du sous-clone de la lignée cellulaire portant de l'ADN du VPH ;
e. l'évaluation de la présence ou de l'absence d'effet inhibiteur du composé sur la maintenance et / ou l'amplification de l'ADN viral dans les cellules ;
f. en cas d'une observation de l'effet inhibiteur sur la réplication de l'ADN du VPH avec une certaine concentration du composé, l'identification du composé en tant que candidat à l'inhibiteur de réplication de l'ADN du VPH.

2. Le procédé selon la revendication 1, dans lequel le composé à l'étude est appliqué à la monocouche de sous-clone de cellule avant d'obtenir une confluence ;et l'inhibiteur du VPH vise à inhiber la phase latente de la réplication de l'ADN du VPH.

3. Le procédé selon l'une quelconque des revendications 1 ou 2, dans lequel la culture du sous-clone est maintenue, au moyen des passages consécutifs, à la confluence pendant au moins 4 à 12 jours jusqu'à ce que la phase de réplication végétative amplificatrice de l'ADN du VPH extrachromosomique sera démarrée, et dans lequel le composé à l'étude est appliqué au milieu de la monocouche de clone cellulaire à la confluence, et dans lequel l'inhibiteur de VPH est testé pour son pouvoir inhibiteur de la phase d'amplification végétative de la réplication de l'ADN du VPH.

4. Le procédé selon l'une quelconque des revendications 1 à 3, dans lequel la présence ou l'absence de l'effet inhibiteur est évaluée en mesurant quantitativement ou semi-quantitativement la quantitéd l'ADN viral extrachromosomique.

5. Le procédé selon l'une quelconque des revendications 1 à 3, dans lequel la séquence d'un gène rapporteur est insérée dans le fragment subgénomique de l'ADN du VPH.

6. Le procédé selon la revendication 5, dans lequel la séquence du gène rapporteur substitue les gènes L1 et L2 du VPH.

7. Le procédé selon la revendication 5, dans lequel le gène rapporteur est d1GFP, luciférase, phosphatase alcaline sécrétée (SEAP) ou luciférase de Gaussia.

8. Le procédé selon la revendication 5, dans lequel la quantité de protéine codée par le gène rapporteur est mesurée.

9. Le procédé selon la revendication 5, dans lequel le produit de la réaction catalysée par la protéine codée par le gène rapporteur est mesuré.

10. Le procédé selon l'une quelconque des revendications 1 à 3, dans lequel le VPH est un VPH muqueux à haut risque appartenant au sous-type VPH-18 ou VPH-16, ou tout autre VPH à grande muqueuse à haut risque.

11. Procédé selon l'une quelconque des revendications 1 à 3, dans lequel le VPH est un VPH muqueux à faible risque appartenant au sous-type VPH-6b ou VPH-11, ou tout autre VPH à la muqueuse à faible risque.

12. Le procédé selon l'une quelconque des revendications 1 à 3, dans lequel le VPH est un type cutané de VPH appartenant au sous-type VPH-5 ou VPH-8, ou tout autre VPH cutané.

13. Kit pour l'identification des composés capables d'inhiber la réplication du VPH lors de la réplication initiale, de la maintenance stable, ou de la phase amplificatrice végétative, ledit kit comprenant au moins :
a) la lignée cellulaire ostéosarcome du système humain U20S permettant la réplication initiale, la maintenance stable, et les phases d'amplification végétative du cycle de réplication de l'ADN du VPH ;
b) la construction extrachromosomiquement durable avec une séquence complète ou partielle de l'ADN duVPH avec des gènes L1 et L2 substitués par des gènes rapporteurs pour l'introduction dans la lignée cellulaire U2OS ;
c) le composé ou une bibliothèque de composés à tester pour une activité anti-VPH ;
d) les moyens pour l'évaluation quantitative de l'activité de réplication, de transcription, ou de translation de l'ADN du VPH dans les cellules.

14. Le kit selon la revendication 13, dans lequel la construction extrachromosomique maintenable est selon la SEQ ID NO:3.


### SEQUENCE LISTING

<110> University of Tartu
   Ustav, Mart
   Ustav, Ene
   Geimanen, Jelizaveta
   Pipit 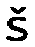 ,Regina
   Isok-Paas, Helen
   Reinson, Tormi
   Ustav, Mart
   Laos, Triin
   Orav, Marit
   Salk, Kristiina
   Männik, Andres
   Remm, Anu
<120> Method and kit for identifying compounds capable of inhibiting Human
   Papilloma virus replication
   <160> 3
   <170> PatentIn Version 3.3
   <210> 1
   <211> 7196
   <212> DNA
   <213> Artificial
   <400> 1
   gtgtgtgtgt atatatatat acatctattg ttgtgtttgt atgtcctgtg tttgtgtttg 60
   ttgtatgatt gcattgtatg gtatgtatgg ttgttgttgt atgttgtatg ttactatatt 120
   tgttggtatg tggcattaaa taaaatatgt tttgtggttc tgtgtgttat gtggttgcgc 180
   cctagtgagt aacaactgta tttgtgtttg tggtatgggt gttgcttgtt gggctatata 240
   ttgtcctgta tttcaagtta taaaactgca caccttacag catccatttt atcctacaat 300
   cctccatttt gctgtgcaac cgatttcggt tgcctttggc ttatgtctgt ggttttctgc 360
   acaatacagt acgctggcac tattgcaaac tttaatcttt tgggcactgc tcctacatat 420
   tttgaacaat tggcgcgcct ctttggcgca tataaggcgc acctggtatt agtcattttc 480
   ctgtccaggt gcgctacaac aattgcttgc ataactatat ccactcccta agtaataaaa 540
   ctgcttttag gcacatattt tagtttgttt ttacttaagc taattgcata cttggcttgt 600
   acaactactt tcatgtccaa cattctgtct acccttaaca tgaactataa tatgactaag 660
   ctgtgcatac atagtttatg caaccgaaat aggttgggca gcacatacta tacttttcat 720
   taatactttt aacaattgta gtatataaaa aagggagtaa ccgaaaacgg tcgggaccga 780
   aaacggtgta tataaaagat gtgagaaaca caccacaata ctatggcgcg ctttgaggat 840
   ccaacacggc gaccctacaa gctacctgat ctgtgcacgg aactgaacac ttcactgcaa 900
   gacatagaaa taacctgtgt atattgcaag acagtattgg aacttacaga ggtatttgaa 960
   tttgcattta aagatttatt tgtggtgtat agagacagta taccccatgc tgcatgccat 1020
   aaatgtatag atttttattc tagaattaga gaattaagac attattcaga ctctgtgtat 1080
   ggagacacat tggaaaaact aactaacact gggttataca atttattaat aaggtgcctg 1140
   cggtgccaga aaccgttgaa tccagcagaa aaacttagac accttaatga aaaacgacga 1200
   tttcacaaca tagctgggca ctatagaggc cagtgccatt cgtgctgcaa ccgagcacga 1260
   caggaacgac tccaacgacg cagagaaaca caagtataat attaagtatg catggaccta 1320
   aggcaacatt gcaagacatt gtattgcatt tagagcccca aaatgaaatt ccggttgacc 1380
   ttctatgtca cgagcaatta agcgactcag aggaagaaaa cgatgaaata gatggagtta 1440
   atcatcaaca tttaccagcc cgacgagccg aaccacaacg tcacacaatg ttgtgtatgt 1500
   gttgtaagtg tgaagccaga attgagctag tagtagaaag ctcagcagac gaccttcgag 1560
   cattccagca gctgtttctg aacaccctgt cctttgtgtg tccgtggtgt gcatcccagc 1620
   agtaagcaac aatggctgat ccagaaggta cagacgggga gggcacgggt tgtaacggct 1680
   ggttttatgt acaagctatt gtagacaaaa aaacaggaga tgtaatatca gatgacgagg 1740
   acgaaaatgc aacagacaca gggtcggata tggtagattt tattgataca caaggaacat 1800
   tttgtgaaca ggcagagcta gagacagcac aggcattgtt ccatgcgcag gaggtccaca 1860
   atgatgcaca agtgttgcat gttttaaaac gaaagtttgc aggaggcagc acagaaaaca 1920
   gtccattagg ggagcggctg gaggtggata cagagttaag tccacggtta caagaaatat 1980
   ctttaaatag tgggcagaaa aaggcaaaaa ggcggctgtt tacaatatca gatagtggct 2040
   atggctgttc tgaagtggaa gcaacacaga ttcaggtaac tacaaatggc gaacatggcg 2100
   gcaatgtatg tagtggcggc agtacggagg ctatagacaa cgggggcaca gagggcaaca 2160
   acagcagtgt agacggtaca agtgacaata gcaatataga aaatgtaaat ccacaatgta 2220
   ccatagcaca attaaaagac ttgttaaaag taaacaataa acaaggagct atgttagcag 2280
   tatttaaaga cacatatggg ctatcattta cagatttagt tagaaatttt aaaagtgata 2340
   aaaccacgtg tacagattgg gttacagcta tatttggagt aaacccaaca atagcagaag 2400
   gatttaaaac actaatacag ccatttatat tatatgccca tattcaatgt ctagactgta 2460
   aatggggagt attaatatta gccctgttgc gttacaaatg tggtaagagt agactaacag 2520
   ttgctaaagg tttaagtacg ttgttacacg tacctgaaac ttgtatgtta attcaaccac     2580
   caaaattgcg aagtagtgtt gcagcactat attggtatag aacaggaata tcaaatatta     2640
   gtgaagtaat gggagacaca cctgagtgga tacaaagact tactattata caacatggaa     2700
   tagatgatag caattttgat ttgtcagaaa tggtacaatg ggcatttgat aatgagctga     2760
   cagatgaaag cgatatggca tttgaatatg ccttattagc agacagcaac agcaatgcag     2820
   ctgccttttt aaaaagcaat tgccaagcta aatatttaaa agattgtgcc acaatgtgca     2880
   aacattatag gcgagcccaa aaacgacaaa tgaatatgtc acagtggata cgatttagat     2940
   gttcaaaaat agatgaaggg ggagattgga gaccaatagt gcaattcctg cgataccaac     3000
   aaatagagtt tataacattt ttaggagcct taaaatcatt tttaaaagga acccccaaaa     3060
   aaaattgttt agtattttgt ggaccagcaa atacaggaaa atcatatttt ggaatgagtt     3120
   ttatacactt tatacaagga gcagtaatat catttgtgaa ttccactagt catttttggt     3180
   tggaaccgtt aacagatact aaggtggcca tgttagatga tgcaacgacc acgtgttgga     3240
   catactttga tacctatatg agaaatgcgt tagatggcaa tccaataagt attgatagaa     3300
   agcacaaacc attaatacaa ctaaaatgtc ctccaatact actaaccaca aatatacatc     3360
   cagcaaagga taatagatgg ccatatttag aaagtagaat aacagtattt gaatttccaa     3420
   atgcatttcc atttgataaa aatggcaatc cagtatatga aataaatgac aaaaattgga     3480
   aatgtttttt tgaaaggaca tggtccagat tagatttgca cgaggaagag gaagatgcag     3540
   acaccgaagg aaaccctttc ggaacgttta agttgcgtgc aggacaaaat catagaccac     3600
   tatgaaaatg acagtaaaga catagacagc caaatacagt attggcaact aatacgttgg     3660
   gaaaatgcaa tattctttgc agcaagggaa catggcatac agacattaaa ccaccaggtg     3720
   gtgccagcct ataacatttc aaaaagtaaa gcacataaag ctattgaact gcaaatggcc     3780
   ctacaaggcc ttgcacaaag tcgatacaaa accgaggatt ggacactgca agacacatgc     3840
   gaggaactat ggaatacaga acctactcac tgctttaaaa aaggtggcca aacagtacaa     3900
   gtatattttg atggcaacaa agacaattgt atgacctatg tagcatggga cagtgtgtat     3960
   tatatgactg atgcaggaac atgggacaaa accgctacct gtgtaagtca caggggattg     4020
   tattatgtaa aggaagggta caacacgttt tatatagaat ttaaaagtga atgtgaaaaa     4080
   tatgggaaca caggtacgtg ggaagtacat tttgggaata atgtaattga ttgtaatgac     4140
   tctatgtgca gtaccagtga cgacacggta tccgctactc agcttgttaa acagctacag     4200
   cacaccccct caccgtattc cagcaccgtg tccgtgggca ccgcaaagac ctacggccag     4260
   acgtcggctg ctacacgacc tggacactgt ggactcgcgg agaagcagca ttgtggacct     4320
   gtcaacccac ttctcggtgc agctacacct acaggcaaca acaaaagacg gaaactctgt     4380
   agtggtaaca ctacgcctat aatacattta aaaggtgaca gaaacagttt aaaatgttta     4440
   cggtacagat tgcgaaaaca tagcgaccac tatagagata tatcatccac ctggcattgg     4500
   acaggtgcag gcaatgaaaa aacaggaata ctgactgtaa cataccatag tgaaacacaa     4560
   agaacaaaat ttttaaatac tgttgcaatt ccagatagtg tacaaatatt ggtgggatac     4620
   atgacaatgt aatacatatg ctgtagtacc aatatgttat cacttatttt tttattttgc     4680
   ttttgtgtat gcatgtatgt gtgctgccat gtcccgcttt tgccatctgt ctgtatgtgt     4740
   gcgtatgcat gggtattggt atttgtgtat attgtggtaa taacgtcccc tgccacagca     4800
   ttcacagtat atgtattttg ttttttattg cccatgttac tattgcatat acatgctata     4860
   ttgtctttac agtaattgta taggttgttt tatacagtgt attgtacatt gtatattttg     4920
   ttttatacct tttatgcttt ttgtattttt gtaataaaag tatggtatcc caccgtgccg     4980
   cacgacgcaa acgggcttcg gtaactgact tatataaaac atgtaaacaa tctggtacat     5040
   gtccacctga tgttgttcct aaggtggagg gcaccacgtt agcagataaa atattgcaat     5100
   ggtcaagcct tggtatattt ttgggtggac ttggcatagg tactggcagt ggtacagggg     5160
   gtcgtacagg gtacattcca ttgggtgggc gttccaatac agtggtggat gttggtccta     5220
   cacgtccccc agtggttatt gaacctgtgg gcccggatcc aagcttacga aagggcctcg     5280
   tgatacgcct atttttatag gttaatgtca tgataataat ggtttcttag acgtcaggtg     5340
   gcacttttcg gggaaatgtg cgcggaaccc ctatttgttt atttttctaa atacattcaa     5400
   atatgtatcc gctcatgaga caataaccct gataaatgct tcaataatat tgaaaaagga     5460
   agagtatgag tattcaacat ttccgtgtcg cccttattcc cttttttgcg gcattttgcc     5520
   ttcctgtttt tgctcaccca gaaacgctgg tgaaagtaaa agatgctgaa gatcagttgg     5580
   gtgcacgagt gggttacatc gaactggatc tcaacagcgg taagatcctt gagagttttc     5640
   gccccgaaga acgttttcca atgatgagca cttttaaagt tctgctatgt ggcgcggtat     5700
   tatcccgtat tgacgccggg caagagcaac tcggtcgccg catacactat tctcagaatg     5760
   acttggttga gtactcacca gtcacagaaa agcatcttac ggatggcatg acagtaagag     5820
   aattatgcag tgctgccata accatgagtg ataacactgc ggccaactta cttctgacaa     5880
   cgatcggagg accgaaggag ctaaccgctt ttttgcacaa catgggggat catgtaactc     5940
   gccttgatcg ttgggaaccg gagctgaatg aagccatacc aaacgacgag cgtgacacca     6000
   cgatgcctgt agcaatggca acaacgttgc gcaaactatt aactggcgaa ctacttactc     6060
   tagcttcccg gcaacaatta atagactgga tggaggcgga taaagttgca ggaccacttc     6120
   tgcgctcggc ccttccggct ggctggttta ttgctgataa atctggagcc ggtgagcgtg     6180
   ggtctcgcgg tatcattgca gcactggggc cagatggtaa gccctcccgt atcgtagtta     6240
   tctacacgac ggggagtcag gcaactatgg atgaacgaaa tagacagatc gctgagatag     6300
   gtgcctcact gattaagcat tggtaactgt cagaccaagt ttactcatat atactttaga     6360
   ttgatttaaa acttcatttt taatttaaaa ggatctaggt gaagatcctt tttgataatc     6420
   tcatgaccaa aatcccttaa cgtgagtttt cgttccactg agcgtcagac cccgtagaaa     6480
   agatcaaagg atcttcttga gatccttttt ttctgcgcgt aatctgctgc ttgcaaacaa     6540
   aaaaaccacc gctaccagcg gtggtttgtt tgccggatca agagctacca actctttttc     6600
   cgaaggtaac tggcttcagc agagcgcaga taccaaatac tgtccttcta gtgtagccgt     6660
   agttaggcca ccacttcaag aactctgtag caccgcctac atacctcgct ctgctaatcc     6720
   tgttaccagt ggctgctgcc agtggcgata agtcgtgtct taccgggttg gactcaagac    6780
   gatagttacc ggataaggcg cagcggtcgg gctgaacggg gggttcgtgc acacagccca    6840
   gcttggagcg aacgacctac accgaactga gatacctaca gcgtgagcta tgagaaagcg    6900
   ccacgcttcc cgaagggaga aaggcggaca ggtatccggt aagcggcagg gtcggaacag    6960
   gagagcgcac gagggagctt ccagggggaa acgcctggta tctttatagt cctgtcgggt    7020
   ttcgccacct ctgacttgag cgtcgatttt tgtgatgctc gtcagggggg cggagcctat    7080
   ggaaaaacgc cagcaacgcg aagcttagat ctcggctagc tagtacttaa ttaacctaag    7140
   gcactacgtc ttctaaacct gccaagcgtg tgcgtgtacg tgccaggaag taatat        7196
<210> 2
   <211> 8230
   <212> DNA
   <213> Artificial
   <400> 2
   gtgtgtgtgt atatatatat acatctattg ttgtgtttgt atgtcctgtg tttgtgtttg 60
   ttgtatgatt gcattgtatg gtatgtatgg ttgttgttgt atgttgtatg ttactatatt 120
   tgttggtatg tggcattaaa taaaatatgt tttgtggttc tgtgtgttat gtggttgcgc 180
   cctagtgagt aacaactgta tttgtgtttg tggtatgggt gttgcttgtt gggctatata 240
   ttgtcctgta tttcaagtta taaaactgca caccttacag catccatttt atcctacaat 300
   cctccatttt gctgtgcaac cgatttcggt tgcctttggc ttatgtctgt ggttttctgc 360
   acaatacagt acgctggcac tattgcaaac tttaatcttt tgggcactgc tcctacatat 420
   tttgaacaat tggcgcgcct ctttggcgca tataaggcgc acctggtatt agtcattttc 480
   ctgtccaggt gcgctacaac aattgcttgc ataactatat ccactcccta agtaataaaa 540
   ctgcttttag gcacatattt tagtttgttt ttacttaagc taattgcata cttggcttgt 600
   acaactactt tcatgtccaa cattctgtct acccttaaca tgaactataa tatgactaag 660
   ctgtgcatac atagtttatg caaccgaaat aggttgggca gcacatacta tacttttcat 720
   taatactttt aacaattgta gtatataaaa aagggagtaa ccgaaaacgg tcgggaccga 780
   aaacggtgta tataaaagat gtgagaaaca caccacaata ctatggcgcg ctttgaggat 840
   ccaacacggc gaccctacaa gctacctgat ctgtgcacgg aactgaacac ttcactgcaa 900
   gacatagaaa taacctgtgt atattgcaag acagtattgg aacttacaga ggtatttgaa 960
   tttgcattta aagatttatt tgtggtgtat agagacagta taccccatgc tgcatgccat 1020
   aaatgtatag atttttattc tagaattaga gaattaagac attattcaga ctctgtgtat 1080
   ggagacacat tggaaaaact aactaacact gggttataca atttattaat aaggtgcctg 1140
   cggtgccaga aaccgttgaa tccagcagaa aaacttagac accttaatga aaaacgacga 1200
   tttcacaaca tagctgggca ctatagaggc cagtgccatt cgtgctgcaa ccgagcacga 1260
   caggaacgac tccaacgacg cagagaaaca caagtataat attaagtatg catggaccta 1320
   aggcaacatt gcaagacatt gtattgcatt tagagcccca aaatgaaatt ccggttgacc 1380
   ttctatgtca cgagcaatta agcgactcag aggaagaaaa cgatgaaata gatggagtta 1440
   atcatcaaca tttaccagcc cgacgagccg aaccacaacg tcacacaatg ttgtgtatgt 1500
   gttgtaagtg tgaagccaga attgagctag tagtagaaag ctcagcagac gaccttcgag 1560
   cattccagca gctgtttctg aacaccctgt cctttgtgtg tccgtggtgt gcatcccagc 1620
   agtaagcaac aatggctgat ccagaaggta cagacgggga gggcacgggt tgtaacggct 1680
   ggttttatgt acaagctatt gtagacaaaa aaacaggaga tgtaatatca gatgacgagg 1740
   acgaaaatgc aacagacaca gggtcggata tggtagattt tattgataca caaggaacat 1800
   tttgtgaaca ggcagagcta gagacagcac aggcattgtt ccatgcgcag gaggtccaca 1860
   atgatgcaca agtgttgcat gttttaaaac gaaagtttgc aggaggcagc acagaaaaca 1920
   gtccattagg ggagcggctg gaggtggata cagagttaag tccacggtta caagaaatat 1980
   ctttaaatag tgggcagaaa aaggcaaaaa ggcggctgtt tacaatatca gatagtggct 2040
   atggctgttc tgaagtggaa gcaacacaga ttcaggtaac tacaaatggc gaacatggcg 2100
   gcaatgtatg tagtggcggc agtacggagg ctatagacaa cgggggcaca gagggcaaca 2160
   acagcagtgt agacggtaca agtgacaata gcaatataga aaatgtaaat ccacaatgta 2220
   ccatagcaca attaaaagac ttgttaaaag taaacaataa acaaggagct atgttagcag 2280
   tatttaaaga cacatatggg ctatcattta cagatttagt tagaaatttt aaaagtgata 2340
   aaaccacgtg tacagattgg gttacagcta tatttggagt aaacccaaca atagcagaag 2400
   gatttaaaac actaatacag ccatttatat tatatgccca tattcaatgt ctagactgta 2460
   aatggggagt attaatatta gccctgttgc gttacaaatg tggtaagagt agactaacag 2520
   ttgctaaagg tttaagtacg ttgttacacg tacctgaaac ttgtatgtta attcaaccac 2580
   caaaattgcg aagtagtgtt gcagcactat attggtatag aacaggaata tcaaatatta 2640
   gtgaagtaat gggagacaca cctgagtgga tacaaagact tactattata caacatggaa 2700
   tagatgatag caattttgat ttgtcagaaa tggtacaatg ggcatttgat aatgagctga 2760
   cagatgaaag cgatatggca tttgaatatg ccttattagc agacagcaac agcaatgcag 2820
   ctgccttttt aaaaagcaat tgccaagcta aatatttaaa agattgtgcc acaatgtgca 2880
   aacattatag gcgagcccaa aaacgacaaa tgaatatgtc acagtggata cgatttagat 2940
   gttcaaaaat agatgaaggg ggagattgga gaccaatagt gcaattcctg cgataccaac 3000
   aaatagagtt tataacattt ttaggagcct taaaatcatt tttaaaagga acccccaaaa 3060
   aaaattgttt agtattttgt ggaccagcaa atacaggaaa atcatatttt ggaatgagtt 3120
   ttatacactt tatacaagga gcagtaatat catttgtgaa ttccactagt catttttggt 3180
   tggaaccgtt aacagatact aaggtggcca tgttagatga tgcaacgacc acgtgttgga 3240
   catactttga tacctatatg agaaatgcgt tagatggcaa tccaataagt attgatagaa 3300
   agcacaaacc attaatacaa ctaaaatgtc ctccaatact actaaccaca aatatacatc    3360
   cagcaaagga taatagatgg ccatatttag aaagtagaat aacagtattt gaatttccaa    3420
   atgcatttcc atttgataaa aatggcaatc cagtatatga aataaatgac aaaaattgga    3480
   aatgtttttt tgaaaggaca tggtccagat tagatttgca cgaggaagag gaagatgcag    3540
   acaccgaagg aaaccctttc ggaacgttta agttgcgtgc aggacaaaat catagaccac    3600
   tatgaaaatg acagtaaaga catagacagc caaatacagt attggcaact aatacgttgg    3660
   gaaaatgcaa tattctttgc agcaagggaa catggcatac agacattaaa ccaccaggtg    3720
   gtgccagcct ataacatttc aaaaagtaaa gcacataaag ctattgaact gcaaatggcc    3780
   ctacaaggcc ttgcacaaag tcgatacaaa accgaggatt ggacactgca agacacatgc    3840
   gaggaactat ggaatacaga acctactcac tgctttaaaa aaggtggcca aacagtacaa    3900
   gtatattttg atggcaacaa agacaattgt atgacctatg tagcatggga cagtgtgtat    3960
   tatatgactg atgcaggaac atgggacaaa accgctacct gtgtaagtca caggggattg    4020
   tattatgtaa aggaagggta caacacgttt tatatagaat ttaaaagtga atgtgaaaaa    4080
   tatgggaaca caggtacgtg ggaagtacat tttgggaata atgtaattga ttgtaatgac    4140
   tctatgtgca gtaccagtga cgacacggta tccgctactc agcttgttaa acagctacag    4200
   cacaccccct caccgtattc cagcaccgtg tccgtgggca ccgcaaagac ctacggccag    4260
   acgtcggctg ctacacgacc tggacactgt ggactcgcgg agaagcagca ttgtggacct    4320
   gtcaacccac ttctcggtgc agctacacct acaggcaaca acaaaagacg gaaactctgt    4380
   agtggtaaca ctacgcctat aatacattta aaaggtgaca gaaacagttt aaaatgttta    4440
   cggtacagat tgcgaaaaca tagcgaccac tatagagata tatcatccac ctggcattgg    4500
   acaggtgcag gcaatgaaaa aacaggaata ctgactgtaa cataccatag tgaaacacaa    4560
   agaacaaaat ttttaaatac tgttgcaatt ccagatagtg tacaaatatt ggtgggatac    4620
   atgacaatgt aatacatatg ctgtagtacc aatatgttat cacttatttt tttattttgc    4680
   ttttgtgtat gcatgtatgt gtgctgccat gtcccgcttt tgccatctgt ctgtatgtgt    4740
   gcgtatgcat gggtattggt atttgtgtat attgtggtaa taacgtcccc tgccacagca    4800
   ttcacagtat atgtattttg ttttttattg cccatgttac tattgcatat acatgctata    4860
   ttgtctttac agtaattgta taggttgttt tatacagtgt attgtacatt gtatattttg    4920
   ttttatacct tttatgcttt ttgtattttt gtaataaaag tatggtatcc caccgtgccg    4980
   cacgacgcaa acgggcttcg gtaactgact tatataaaac atgtaaacaa tctggtacat    5040
   gtccacctga tgttgttcct aaggtggagg gcaccacgtt agcagataaa atattgcaat    5100
   ggtcaagcct tggtatattt ttgggtggac ttggcatagg tactggcagt ggtacagggg    5160
   gtcgtacagg gtacattcca ttgggtgggc gttccaatac agtggtggat gttggtccta    5220
   cacgtccccc agtggttatt gaacctgtgg gcccggatcc aagcttacga aagggcctcg    5280
   tgatacgcct atttttatag gttaatgtca tgataataat ggtttcttag acgtcaggtg    5340
   gcacttttcg gggaaatgtg cgcggaaccc ctatttgttt atttttctaa atacattcaa    5400
   atatgtatcc gctcatgaga caataaccct gataaatgct tcaataatat tgaaaaagga    5460
   agagtatgag tattcaacat ttccgtgtcg cccttattcc cttttttgcg gcattttgcc    5520
   ttcctgtttt tgctcaccca gaaacgctgg tgaaagtaaa agatgctgaa gatcagttgg    5580
   gtgcacgagt gggttacatc gaactggatc tcaacagcgg taagatcctt gagagttttc    5640
   gccccgaaga acgttttcca atgatgagca cttttaaagt tctgctatgt ggcgcggtat    5700
   tatcccgtat tgacgccggg caagagcaac tcggtcgccg catacactat tctcagaatg    5760
   acttggttga gtactcacca gtcacagaaa agcatcttac ggatggcatg acagtaagag    5820
   aattatgcag tgctgccata accatgagtg ataacactgc ggccaactta cttctgacaa    5880
   cgatcggagg accgaaggag ctaaccgctt ttttgcacaa catgggggat catgtaactc    5940
   gccttgatcg ttgggaaccg gagctgaatg aagccatacc aaacgacgag cgtgacacca    6000
   cgatgcctgt agcaatggca acaacgttgc gcaaactatt aactggcgaa ctacttactc    6060
   tagcttcccg gcaacaatta atagactgga tggaggcgga taaagttgca ggaccacttc    6120
   tgcgctcggc ccttccggct ggctggttta ttgctgataa atctggagcc ggtgagcgtg    6180
   ggtctcgcgg tatcattgca gcactggggc cagatggtaa gccctcccgt atcgtagtta    6240
   tctacacgac ggggagtcag gcaactatgg atgaacgaaa tagacagatc gctgagatag    6300
   gtgcctcact gattaagcat tggtaactgt cagaccaagt ttactcatat atactttaga    6360
   ttgatttaaa acttcatttt taatttaaaa ggatctaggt gaagatcctt tttgataatc    6420
   tcatgaccaa aatcccttaa cgtgagtttt cgttccactg agcgtcagac cccgtagaaa    6480
   agatcaaagg atcttcttga gatccttttt ttctgcgcgt aatctgctgc ttgcaaacaa    6540
   aaaaaccacc gctaccagcg gtggtttgtt tgccggatca agagctacca actctttttc    6600
   cgaaggtaac tggcttcagc agagcgcaga taccaaatac tgtccttcta gtgtagccgt    6660
   agttaggcca ccacttcaag aactctgtag caccgcctac atacctcgct ctgctaatcc    6720
   tgttaccagt ggctgctgcc agtggcgata agtcgtgtct taccgggttg gactcaagac    6780
   gatagttacc ggataaggcg cagcggtcgg gctgaacggg gggttcgtgc acacagccca    6840
   gcttggagcg aacgacctac accgaactga gatacctaca gcgtgagcta tgagaaagcg    6900
   ccacgcttcc cgaagggaga aaggcggaca ggtatccggt aagcggcagg gtcggaacag    6960
   gagagcgcac gagggagctt ccagggggaa acgcctggta tctttatagt cctgtcgggt    7020
   ttcgccacct ctgacttgag cgtcgatttt tgtgatgctc gtcagggggg cggagcctat    7080
   ggaaaaacgc cagcaacgcg aagcttagat ctcggctagc gtatacggat cgatcctgca    7140
   ggtcgactct agacaggtaa gtggcgtttc tcggggagcc agctgcgtcc gctgtcgtgc    7200
   tgtcggtgta gtactagcaa gcgttaagtc cccatctggc tgcggcctac cgaagagtgg    7260
   tcttcacgtc acacgctgtc ccacgcacgt ggttggtttg gtcgcttctg gttactgact    7320
   actaagcagc cttttctttt ttcctttcag gttctagacg ccaccatggg cgtgaaggtg    7380
   ctgttcgccc tgatctgtat cgccgtggcc gaggccaagc ccaccgagaa caacgaggac    7440
   ttcaacatcg tggccgtggc cagcaacttc gccaccacag acctggacgc cgacagaggc    7500
   aagctgcccg gcaagaaact gcccctggaa gtgctgaaag agatggaagc caacgccaga    7560
   aaggccggct gcaccagagg ctgcctgatc tgcctgagcc acatcaagtg cacccccaag    7620
   atgaagaagt tcatccccgg cagatgccac acctacgagg gcgacaaaga gagcgcccag    7680
   ggcggcatcg gcgaggccat cgtggacatc cccgagatcc ccggcttcaa ggacctggaa    7740
   cccatggaac agtttatcgc ccaggtggac ctgtgcgtgg actgcaccac cggctgtctg    7800
   aagggcctgg ccaacgtgca gtgcagcgac ctgctgaaga agtggctgcc ccagagatgc    7860
   gccaccttcg ccagcaagat ccagggccag gtggacaaga tcaagggcgc tggcggcgac    7920
   tgatgagcgg ccgcctcgag ctcgctgatc agcctcgact gtgccttcta gttgccagcc    7980
   atctgttgtt tgcccctccc ccgtgccttc cttgaccctg gaaggtgcca ctcccactgt    8040
   cctttcctaa taaaatgagg aaattgcatc gcattgtctg agtaggtgtc attctattct    8100
   ggggggtggg gtggggcagg acagcaaggg ggaggattgg gaagacaata gcaggcatgc    8160
   ttaattaacc taaggcacta cgtcttctaa acctgccaag cgtgtgcgtg tacgtgccag    8220
   gaagtaatat                                                          8230
<210> 1
   <211> 8983
   <212> DNA
   <213> Artificial
   <400> 3